# EUROPEAN PATENT APPLICATION

(11) **EP 4 353 307 A1**
(43) Date of publication of application: **17.04.2024**
(21) Application number: 22806895.3
(22) Date of filing: 13.05.2022
(51) Int. Cl.: A61N 5/06

(54) **LIGHTING DEVICE WITH ORIENTED LED DIODES**

(30) Priority: 14.05.2021 ES 202130442
(71) Applicant: Universidad De Malaga, E-29071 Málaga (ES); Universidad de Alcalá, 28801 Alcala de Henares (Madrid) (ES); Servicio Andaluz de Salud, 41071 Sevilla (ES)
(72) Inventor: NAVARRETE DE GÁLVEZ, Enrique, 29590 Málaga (ES); AGUILERA ARJONA, José, 29010 Málaga (ES); FONDA PASCUAL, Pablo, 28805 Alcalá de Henares (MADRID) (ES); DE GÁLVEZ ARANDA, María Victoria, 29010 Málaga (ES); GAGO CALDERÓN, Alfonso, 29010 Málaga (ES); HERRERA CEBALLOS, Enrique, 29010 Málaga (ES); DE ANDRÉS DÍAZ, José Ramón, 29590 Málaga (ES); VIDAL ASENSI, Santiago, 28805 Alcalá de Henares (Madrid) (ES)
(74) Representative: Hoffmann Eitle
(86) International application number: PCT/ES2022/070297
(87) International publication number: WO 2022/238609

(57) **Abstract**

The present invention relates to a lighting device comprising a plurality of LED diodes (1), installed and distributed on at least one support arm (2), all the LED diodes (1) being oriented towards a single treatment point (3) and the emitted light (8) of said diodes being focussed towards said treatment point (3) by means of light-concentrating means (4). In this manner, it is possible to carry out localised treatments of photodynamic therapy, without affecting the healthy surface, adapting the dose to the requirements of the lesioned surface.

## Description

### OBJECT OF THE INVENTION

The object of the present patent application relates to a lighting device comprising a plurality of LED diodes installed and distributed on at least one support arm, all the LED diodes being oriented towards a single treatment point and the emitted light of said diodes being focussed towards said treatment point by means of light-concentrating means, incorporating significant innovations and advantages.

### BACKGROUND OF THE INVENTION

A number of light therapies are currently available for the treatment of a variety of dermatological lesions, such as some types of cancers, wounds, infections, mycosis, etc. Said therapies also have applications in the field of skin cosmetics. Techniques of this type are known as photodynamic therapies (PDT). Indirect photodynamic therapy is a treatment that consists of applying a photosensitising compound to a lesion or area to be treated. The compound is taken up by the target cells and metabolised into a substance sensitive to certain ranges of the visible spectrum. When the lesion is illuminated with a source radiating in one of the sensitive ranges, at certain wavelengths, a photochemical reaction takes place, which ends in cell death by apoptosis when the biological substrate oxidised by singlet oxygen is the mitochondrial membrane, or necrosis when the oxidised membrane is the plasma membrane. Conventional treatment therapies, by means of topical, systemic, and combination products, although achieving good results, are not without adverse side effects. Photodynamic therapy is a safe treatment which does not generate cumulative toxicity and does not produce severe side effects, the most representative side effect being pain, which can be treated with analgesia.

Lighting systems commonly used in photodynamic treatment units are laser light sources, conventional LED lamps and luminaires. In the case of conventional lamps that radiate a more or less continuous light spectrum, filters are required to achieve the desired treatment spectrum, which involves a loss of efficiency and lack of precision.

This represents an increased complexity. Luminaires of this type normally require a support structure for positioning on the area to be treated. In the case of LED luminaires, they are one or more panels for the treatment of large surfaces. The problem arising with emitters of this type, in addition to the complexity of their design, size, and weight, which makes it necessary to use a suitable support structure with multiple articulations, is the impossibility of carrying out localised treatments without affecting a healthy surface, or a lesioned surface but with different treatment needs, a drawback which is shared with the previous technology. In these cases, nearby small lesions cannot adapt the dose to the required dose, resulting in overexposure or underexposure, depending on the case. This problem means that for localised treatments, technologies such as lasers are used to cure lesions of this type. An example of this situation would be nail psoriasis, where the lesion is covered by the nail; each nail has a different thickness and, accordingly, a different transmittance of incident radiation. In cases such as this, the dose needs to be corrected according to the lesion to be treated and a specific treatment needs to be given for each lesion. Despite being able to adapt the dose with the use of the therapeutic laser, said therapeutic laser usually works in wavelengths corresponding to the red and infrared range, which does not usually coincide with the most effective peak for stimulating the photosensitiser induced by the photosensitising compound.

On the other hand, a photodynamic therapeutic device for treating cancer is known from the prior art as described in document CA2775660. The therapeutic device comprises at least one light source that can be coupled to a patient's body, adapted to provide an effective light flux to a lesioned area. The therapeutic device further comprises an annular structure that surrounds the patient's body and a passage connectable to a cooling circuit that provides a circulation of coolant in the passage. The light source is fixed to an inner surface of the annular structure. The coolant housed in the passage is suitable for removing the heat generated by the light source.

An LED light therapy device capable of irradiating light onto facial skin and body skin, regardless of the position and posture, is also known from the prior art as described in document KR20200144444. It includes a red or deep red output of an LED light source unit having a plurality of wavelength ranges. It has an LED light source module, a light irradiation unit with a lateral irradiation surface inclined at an angle with respect to the central irradiation surface, and a support mechanism, all forming an LED light therapy device.

In view of the above, the need for having a lighting device that makes it possible to carry out localised treatments of photodynamic therapy, without affecting the healthy surface, adapting the dose to the requirements of the lesioned surface, can be seen.

### DESCRIPTION OF THE INVENTION

The present invention consists of a lighting device, preferably intended for photodynamic therapy, although it would likewise be applicable for phototherapy, and also for diagnosis, selecting the LED diodes according to the wavelengths required according to the intended application or use.

In terms of the preferred approach to photodynamic therapy, the lighting device is of particular application in the photodynamic treatment of dermatological lesions such as cancers, wounds, infections, mycosis, etc.

One of the main tools for treatments of this type are monochromatic luminaires capable of working at different wavelengths with relatively large treatment surfaces. This makes it possible to treat different pathologies by selecting the most appropriate wavelengths from those available and the time needed depending on the dose required. However, for optimal treatment, it is necessary for the wavelengths radiated by the source to coincide with the most sensitive wavelengths of a photosensitive substance metabolised by applying a compound or drug on the patient. The aim is also for treatment times to be as short as possible, and for the treatment to be carried out over the entire thickness affected by the lesion, i.e., the epidermis, dermis and hypodermis, and for it to be limited to the lesioned area, allowing the dose to be adjusted without the need to overexpose or underexpose areas adjacent to the treated area.

More particularly, the lighting device comprises a plurality of LED diodes, wherein said LED diodes are installed and distributed on at least one support arm, all the LED diodes being oriented towards a single treatment point and the emitted light of said diodes being focussed towards said treatment point by means of light-concentrating means. In this manner, and by means of the geometric configuration adopted together with the presence of the light-concentrating means, all the light radiation from the plurality of LED diodes is focussed on a small region of space coinciding with the diagnostic or treatment point of the patient. As the resulting lighting device is small-sized and lightweight, it allows comfortable handling and easy orientation. Optionally, it can be coupled to a universal laboratory stand and/or clamp in order to fix the distance to the patient's treatment or diagnostic point with greater precision and stability.

Note that this lighting device can be used in treatments, or in the establishment of protocols, of photodynamic therapy, or also to evaluate the sensitivity of the skin to different wavelengths, either in a healthy individual or in a photosensitive individual, in any range of the visible spectrum by simply arranging the suitable LED diodes. Alternatively, it can also be used to diagnose conditions, for example skin conditions.

In a preferred embodiment of the invention, the lighting device comprises a plurality of support arms, with at least one LED diode on each one, such that the distribution of the LED diodes is more versatile, being able to adopt a broader and more varied geometric configuration, in order to optimise the results of the treatment or diagnosis. In the event that the support arms have a function of resting on the patient's body, the presence of a plurality of support arms provides greater stability in the positioning and therefore in the application of light radiation.

Advantageously, the lighting device comprises a plurality of LEDs on each of the support arms, such that it is possible to deliver a higher dose of light radiation per unit time, reducing the treatment time required to administer a given dose.

Preferably, the support arms are installed on holding means, which allows the user to hold and apply the lighting device. In a preferred embodiment, they are installed in an articulated manner, which allows folding the support arms and facilitating their folding, collection, storage, etc.

In a preferred embodiment of the invention, the lighting device comprises three support arms arranged with an orientation of 120° with respect to one other, considering each support arm to be contained in a vertical plane, which preferably passes over the axis of the holding means, which are optionally cylindrical in shape. The fact that the three support arms are arranged at 120° determines that the three support arms and the respective LED diodes are equidistant from each other, emitting a more uniform light radiation. On the other hand, a more stable positioning can be achieved by means of resting the ends of the support arms on the body of the patient to be treated.

More specifically, the LED diodes are installed on each support arm by means of a removable and therefore replaceable intermediate array. Greater flexibility is thereby obtained in the placement of LED diodes, of one type or another, in one quantity or another. Thus, the treatment light is obtained as one of the possible combinations of lights emitted by each intermediate array, one by one, two by two or three by three.

Optionally, said intermediate array comprises a plurality of planes on which each of the LED diodes is fixed, by way of a stable support, which prevents the oscillation and swaying of the light emitted by said LED diodes.

According to another aspect of the invention, the planes of the intermediate array are arranged with a fixed orientation of 160° with respect to one another, such that the light emitted by all the LED diodes located on the planes converges towards a single point in space, coinciding with the treatment point.

In a complementary manner, each support arm comprises a plurality of segments with a fixed orientation of 160° with respect to one another, on which segments the planes of the intermediate array are seated, such that a better fit and greater stability between the support arm and the intermediate array are produced. Optionally, the segments are preferably rectilinear and oriented such that the support arm has an overall substantially arcuate shape.

According to another aspect of the invention, each support arm comprises a plurality of segments attached to one another by means of articulations, wherein each plane of the intermediate array is seated on each of the segments. In this manner, the relative orientation of each segment can be adjusted, configured for a larger or smaller distance, orientation, folding, etc. In this embodiment, the intermediate array can have certain flexibility in the attachment between the planes thereof so as to be adapted to the relative orientation adopted by each segment.

Note that the at least one support arm of the lighting device is made of aluminium, such that the support arm itself acts as a heat sink.

Moreover, note that the light-concentrating means of the lighting device comprise at least one collimating lens for the light emitted by each LED diode. In this manner, all the light emitted by the plurality of LED diodes is focussed on a small region of space coinciding with the treatment point. "Collimating" is understood to mean the action or effect whereby a system obtains a parallel beam from a divergent beam. Note that the LED diodes themselves emit a hemispherical light radiation.

Preferably, each LED diode is installed on a printed circuit board including electrical connection means and fixing means for the electrical connection and fixing to each plane of the intermediate array, such that it can be supplied with power and positioned correctly in a continuous manner.

Additionally, the lighting device comprises a power source and control means for the power supply to the LED diodes, such that the emitted light radiation and operating time can be adjusted with precision. The power supply can be either a block supply to each array with LED diodes or an individual supply to each LED diode, with the possibility of switching on not per array but per individual LED diode, increasing versatility but without increasing the complexity.

More specifically, the control means are configured to determine the combination of LED diodes to be activated in a specific sequence of times, preferably depending on the photosensitising compound or drug administered. Said control allows adjusting the treatment light and doses depending on the body part, on the type of dermatological lesion and on the thickness thereof.

More specifically, the lighting device comprises an interface for the user with adjustment means for adjusting the configuration of the control means, for better handling by the user. Said interface comprises at least one display, optionally a touch screen, and in an alternative or complementary manner, a keypad, through which the configuration parameters are input.

Advantageously, the LED diodes are high-power LED diodes for a more efficient application of light radiation doses. An LED diode the power of which is greater than 0.5 W is considered a high-power LED diode.

Note that in this regard, the use of high-power LED diodes allows the configuration of precise spectra which adapt to the treatment spectrum required by the photosensitiser and reach the full treatment thickness, i.e., epidermis, dermis and hypodermis. LED technology allows selecting emitters with a very narrow emission spectrum with a central peak at a desired wavelength, and therefore a spectrum devoid of peaks such as those that could be obtained with fluorescent light. Furthermore, the irradiance emitted by high-power diodes is high, reducing the times to reach the desired doses. They allow the generation of arrays with any type of geometry. It is possible to add accessories, such as lenses, thereto to improve their efficiency in a specific application. They can be controlled individually or in groups, with stable spectra that do not require continuous calibration. All these features make them ideal for the development of a low-cost, easyto-use photodynamic therapy treatment luminaire.

In a preferred embodiment of the invention, the lighting device comprises at least one first LED diode emitting light in a first wavelength, said first wavelength substantially coinciding with at least one more sensitive first wavelength of a photosensitiser induced in a patient by at least one photosensitising compound. The light thereby provides an increased therapeutic effect on a localised area, reducing the necessary application time.

Note that the lighting device of the present invention is preferably intended for photodynamic therapy of dermatological lesions in a patient who has previously been administered a chemical compound or drug that causes a photosensitising effect in the patient's treatment area, specifically the application of a photosensitising substance on the patient's dermatological lesion.

Additionally, the lighting device comprises at least one second LED diode emitting light in a second wavelength different from the first wavelength of the first LED diode, said second wavelength substantially coinciding with at least one more sensitive second wavelength of a photosensitiser induced in a patient by at least one photosensitising compound. A photodynamic therapy effect is thereby achieved by means of highly irradiated monochromatic light obtained by combining several monochromatic spectra on one and the same treatment point. The treatment light is obtained as one of the possible combinations of light emitted by the LED diodes of each intermediate array.

It should be emphasised that the present invention allows a localised treatment of the lesion without affecting healthy adjacent areas or areas with other needs, reaching the epidermis, dermis and hypodermis as a result of the use of radiation of different wavelengths, which are also the most effective in the stimulation of porphyrins. Similarly, instead of using sequential radiation by wavelength, it is possible to use combined radiation.

The therapeutic method could follow the following steps: i) once the thickness is known, the lesion is prepared by applying the suitable compound or drug; ii) waiting for the drug to fix; iii) cleaning the affected area and applying the radiation at the programmed dose. The adjustment means select, based on the photosensitiser used, the percentages of radiation at each of the available wavelengths of the device, in order to limit the treatment to the lesion, increase efficiency and reduce the times to receive the therapeutic dose. Therefore, the present invention allows localised treatment of the lesion without affecting adjacent healthy areas or areas with other needs, and in the precise thickness as a result of the use of radiation of different wavelengths, which are furthermore the most effective in the stimulation of porphyrins.

The attached drawings show, by way of non-limiting example, a lighting device constituted in accordance with the invention. Other features and advantages of the lighting device object of the present invention will become apparent from the description of a preferred, but not exclusive, embodiment which is illustrated by way of non-limiting example in the accompanying drawings.

### BRIEF DESCRIPTION OF THE DRAWINGS

Figure 1 shows a general view of the lighting device, with an elevational, plan and profile view of the support arm and the plurality of LED diodes, according to the present invention;
Figure 2 shows a detailed profile view of the support arm, intermediate array and the plurality of LED diodes, according to the present invention;
Figure 3 shows a detailed view of a patient with a skin lesion to be treated according to the present invention;
Figure 4 shows a detailed view of a patient being treated with a lighting device according to the present invention;
Figure 5 shows a detailed view of the relative spectral irradiance per LED diode according to the present invention;
Figure 6 shows a detailed view of the absolute irradiance of the lighting device in a particular embodiment with three arms according to the present invention;
Figure 7 shows a detailed view of overlapping absorption spectra of different photosensitisers commonly used in therapy and in research with lamp emission spectrum in a particular embodiment according to the present invention;
Figure 8 shows a view of a table of photosensitising compounds in photodynamic therapy (PDT) according to the present invention;

### DESCRIPTION OF A PREFERRED EMBODIMENT

In view of the mentioned figures and according to the numbering adopted, a preferred embodiment of the invention, comprising the parts and elements indicated and described in detail below can be seen in them.

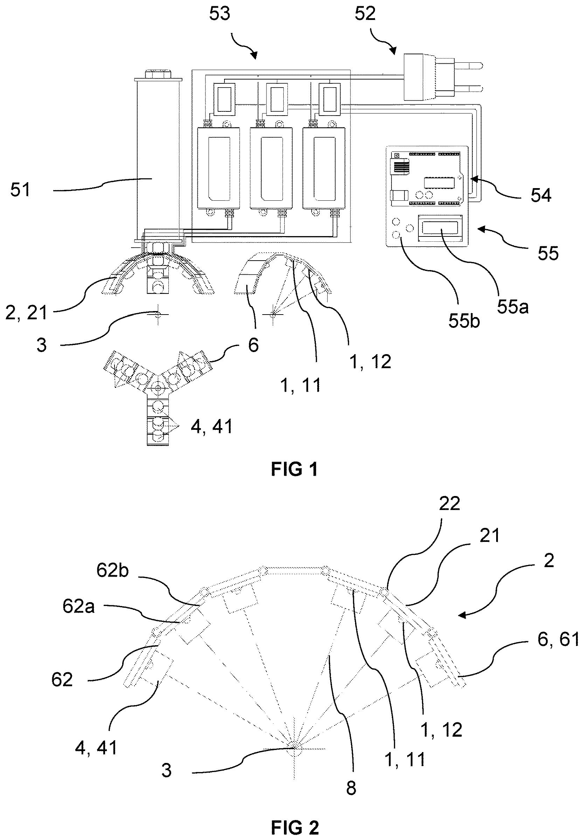

As can be observed in Figure 1, which corresponds to a preferred embodiment of the invention, the lighting device comprises a plurality of LED diodes (1) distributed on support arms (2) and focussed towards a treatment point (3), the emitted light passing through light-concentrating means (4). Holding means (51) for better handling by the user (9) can also be observed. Moreover, it includes a power source (52), the supply of which is controlled by control means (53) and adjustment means (54), specifically an interface (55) with a display (55a) and a keypad (55b).

More specifically, the support arms (2) are attached to the holding means (51) by an assembly screw, a locking washer and a positioning self-locking nut. Said holding means (51) are commonly made of an insulating plastic material. Moreover, actuators or drivers for controlling each LED diode (1) or plurality of LED diodes (1) of each intermediate array (6) can be seen. It also preferably includes at least one on/off relay of each group of LED diodes (1) connected with the control means (53). It also has AC voltage singlephase grid supply wiring (230 V - 50 Hz) and a low-voltage grid connection plug (230 V - 50 Hz).

As can be observed in said preferred embodiment of Figure 1, each intermediate array (6) of each support arm (2) with its LED diodes (1) is electrically powered independently. Each group of LED diodes (1) emits light in one wavelength. The geometric configuration concentrates the light radiated by each emitter in a specific region in space corresponding to the treatment point (3). Said particular embodiment has three intermediate arrays (6) each made up of three printed circuits or boards (62) connected in series, each one capable of having a single high-power LED diode (1).

It also has a series of collimating lenses (41) for concentrating the energy radiated by each LED diode (1), at a different angle, on the surface to be treated. Therefore, the invention allows adjusting the treatment surface in the range of 0.1 - 25 cm², which corresponds to a circle of 0.4 - 5.7 cm in diameter.

As indicated, in said particular embodiment of Figure 1, there are three power supplies, one per intermediate array (6) with its LED diodes (1), which allows independent control of each group. For this purpose, there are three relays, one per power supply, which will act as on/off switches for each of them according to the instructions received from the adjustment means (54) to which they are connected. Said adjustment means (54) are programmed or configured to determine a combination of LED diodes (1) to be activated, in specific sequences and times, depending on the photosensitising drug compound used for treatment, the part of the body, the type of lesion and the thickness. The interface (55) comprises a display (55a), optionally a touch screen, and, in a complementary manner, a keypad (55b), for inputting parameters in the adjustment means (54).

According to another aspect of the invention, in the view of Figure 1, the lighting device comprises holding means (51) for holding and handling the device, attached in this case to a multiple support arm (2) made of aluminium acting as a heat sink, and used for positioning PCB boards (62), light emitters or LED diodes (1), and collimating lenses (41), and the geometric configuration of which focusses the irradiance on a point in space coinciding with the treatment point (3). The holding means (51) form the main body of the equipment with a dimension of 15 - 20 cm in length and 3 - 4 cm in diameter, being made of an insulating plastic material.

Figure 1 shows a preferred embodiment, with a geometric configuration structured in three support arms (2), arranged at an angle of 120° with respect to one another, including three segments (21) in each arm at an inclination of 160° with respect to one another, in order to concentrate the light flux in a specific region in space, referred to as treatment point (3). The lighting device can optionally incorporate more support arms (2), preferably at regular angles: three arms at 120°, four arms at 90°, five arms at 72°, etc.

Further note that in this embodiment of Figure 1, there is arranged on each of the support arms (2) its respective intermediate array (6) configured by three individual PCB boards (62) connected to one another in series, which are fixed to each of the segments (21) that each of the support arms (2) has. Three sources with constant intensity, one per intermediate array (6), with independent on/off control for each of them by means of switches operated by the adjustment means (54) which turns the power to each source on or off, are included. The adjustment means (54) will act by opening or closing the switches powering each intermediate array depending on the program or configuration thereof.

As can be observed in Figure 2, the support arm (2) comprises, in said embodiment, a series of segments (21) and articulations (22) which enable the angular adjustment of the geometric configuration, both of the support arm (2) and of the intermediate array (6). It can be understood from Figure 2 that the planes (61) of said intermediate array (6) are likewise articulated, each of them housing its corresponding board (62) with its electrical connection means (62a) and fixing means (62b) for the respective LED diode (1) and collimating lens (41).

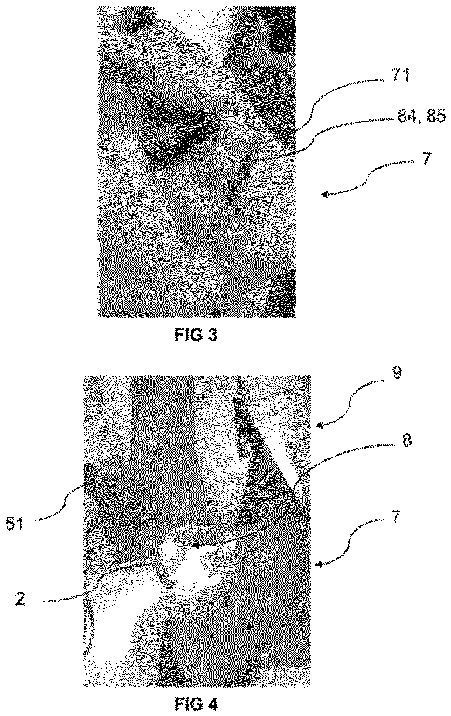

Figure 3 shows a patient (7) with a dermatological lesion (71) to be treated, having administered to said patient a photosensitising compound (85) which generates a photosensitising element (84).

As can be observed in Figure 4, the user (9) can easily handle the lighting device, gripping it by the handle or holding means (51) when applying light treatment (8) to the patient (7).

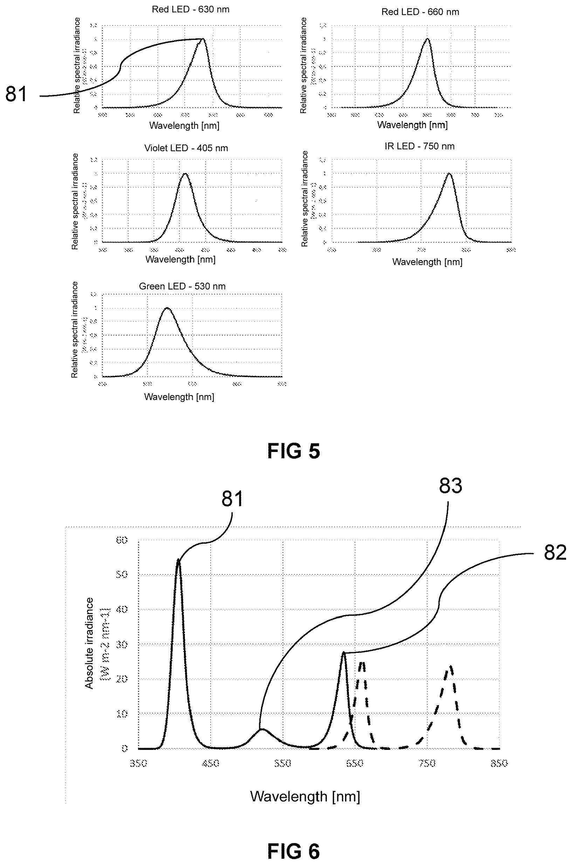

Figure 5 shows a series of graphs of the relative spectral irradiance per LED diode (1), specifically the violet (405 nm), green (530 nm), red (630 nm), red (660 nm), infrared (790 nm) spectra, which are usually selected for photobiological treatments.

Figure 6 shows a graph of the absolute irradiance of the lighting device in a particular embodiment with three support arms (2), showing the peaks in a first wavelength (81), a second wavelength (82) and a third wavelength (83). Specifically, the continuous line shows the absolute irradiance of the violet (405 nm), green (530 nm), red (630 nm) LED diode device (1) at the treatment distance. The discontinuous line shows the absolute irradiance at the treatment distance of an intermediate array (6) of red (660 nm) light (8), and an intermediate array (6) of infrared (790 nm) light (8).

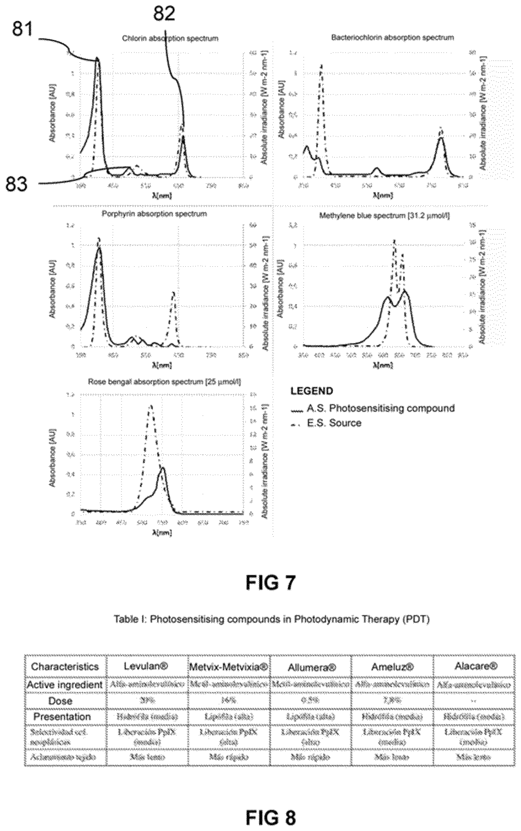

Figure 7 shows a graph with the detail of overlapping absorption spectra of different photosensitisers commonly used in therapy and in research with a lamp or lighting device emission spectrum, showing the peaks in a first wavelength (81), a second wavelength (82) and a third wavelength (83) for various photosensitising substances or compounds (85).

By way of illustration, in the case of a photosensitiser of the chlorin group, it would have three support arms (2) with violet (405 nm), green (530 nm) and red (660 nm) LED diodes (1). For bacteriochlorins, it would have two support arms (2) with violet (405 nm) and IR (790 nm) LED diodes (1). For porphyrins, it would have three support arms (2) with violet (405 nm), green (530 nm) and red (630 nm) LED diodes (1). For methylene blue, at a preferred concentration of 31.2 µmol/L, it would have two support arms (2) with red (630 nm) and red (660 nm) LED diodes (1). And for rose bengal, it would have three support arms (2) with green (530 nm), green (530 nm) and green (530 nm) LED diodes (1).

Figure 8 includes a table showing photosensitising compounds (85) preferred in photodynamic therapy (PDT).

More particularly, and as observed in Figure 1, the lighting device comprises a plurality of LED diodes (1), installed and distributed on at least one support arm (2), all the LED diodes (1) being oriented towards a single treatment point (3), and the emitted light (8) of said diodes being focussed towards said treatment point (3) by means of light-concentrating means (4). Note that the lighting device can be positioned by the user (9) at the treatment distance by simply approaching or separating the device with respect to the lesion. At the treatment point (3), the light beams radiated at a single point (circle of 0.5 - 0.6 cm and surface of 0.2 - 0.3 cm²) are superimposed.

In a particular embodiment, it can be applied for 0.1 cm² point treatments, which corresponds to a circle of 0.4 cm in diameter, which would be a good definition, and surface treatments of 25 cm², which corresponds to a circle of 5.7 cm in diameter. In another particular embodiment of this second object, the design of the invention requires a distance to the treatment point (3) of 5 - 6 cm.

Additionally, as observed in Figure 1, the lighting device comprises a plurality of support arms (2), with at least one LED diode (1) on each one.

In further detail, as observed in Figures 1 and 2, the lighting device comprises a plurality of LED diodes (1) on each of the support arms (2).

Note that, as observed in Figure 1, the support arms (2) are installed on holding means (51), this preferably being a main body of the equipment an arm of 15 - 20 cm in length and 3 - 4 cm in diameter, made of an insulating plastic material.

Moreover, note that, as observed in Figure 1, the lighting device comprises three support arms (2), arranged with an orientation of 120° with respect to one another.

A specific geometry could be that of three support arms (2) of 7.4 - 7.6 cm in length, arranged at 120°, curved inwardly at regular distances in three segments (21) per support arm (2), with an angle of inclination of each segment (21) with respect to the previous one of 160°. This particular embodiment concentrates the treatment light (8) at a distance, as mentioned, of between 5 and 6 cm and on a localised lesion of 0.2 - 0.3 cm², which corresponds to a circle of 0.5 - 0.6 cm in diameter.

And due to the possibility of the lighting device incorporating more arms at regular angles (three arms at 120°, four arms at 90°, five arms at 72°, etc.) without this representing a conceptual variation of the invention, the equipment allows being customised in those cases where it is required for photosensitising compounds (85) with more than three peaks in their absorption spectrum.

Preferably, as observed in Figure 1, the LED diodes (1) are installed on each support arm (2) by means of a removable intermediate array (6).

More specifically, as observed in Figure 2, the intermediate array (6) comprises a plurality of planes (61) on which each of the LED diodes (1) is fixed.

Optionally, as observed in Figure 2, the planes (61) of the intermediate array (6) are arranged with a fixed orientation of 160° with respect to one another.

Note that in a preferred embodiment, the support arm (2), which acts as a heat sink, is fixed to the lower part of the main body. Said support arm (2) on which the remaining elements of the assembly are installed has a thickness of 2.5 - 3 mm and a width of 2 - 2.5 cm. The support arm (2) is curved inwardly in three segments (21) of 2 - 2.5 cm in width by 2 - 2.5 cm in length.

More specifically, as observed in Figure 2, each support arm (2) comprises a plurality of segments (21) with a fixed orientation of 160° with respect to one another, on which the planes (61) of the intermediate array (6) are seated. Said geometry is the one that causes the perpendicular of each segment (21) to intersect and pass through a single point in space, the treatment point (3).

According to another aspect of the invention, as observed in Figure 2, each support arm (2) comprises a plurality of segments (21) attached to one another by means of articulations (22), wherein each plane (61) of the intermediate array (6) is seated on each of the segments (21).

Optionally, as observed in Figure 1, at least one support arm (2) is made of aluminium.

And as observed in Figures 1 and 2, the light-concentrating means (4) comprise at least one collimating lens (41) for the emitted light (8) emitted by each LED diode (1). It would be possible to use collimating lenses having a different angle, and thereby adjust the treatment surface in the range of 0.1 - 25 cm², which corresponds to a circle of 0.4 - 5.7 cm in diameter.

According to another aspect of the invention, as observed in Figure 2, each LED diode (1) is installed on a printed circuit board (62) including electrical connection means (62a) and fixing means (62b) for the electrical connection and fixing to each plane (61) of the intermediate array (6). In a preferred embodiment, each intermediate array (6) houses three independent printed circuit boards (62) connected in series, each of which has an LED diode (1) of the same wavelength, there being three intermediate arrays (6) with three LED diodes (1) each for each support arm (2).

In further detail, as observed in Figure 2, the LED diodes (1) are high-power LED diodes. Each high-power LED diode (1) is installed on a printed circuit board (62) having positive/negative power supply connections for a single LED diode (1), and two boreholes for the fixing thereof.

In a preferred embodiment of the invention, as observed in Figures 4 and 7, the lighting device comprises at least one first LED diode (11) emitting light (8) in a first wavelength (81), said first wavelength (81) substantially coinciding with at least one more sensitive first wavelength (81) of a photosensitiser (84) induced in a patient (7) by at least one photosensitising compound (85). 5-aminolevulinic acid (5-ALA) and derivatives thereof are commonly used as precursors of photosensitisers. These compounds cause the synthesis of porphyrins with absorption spectra showing peak values at 409, 509, 544, 548 and 634 nm. The absolute peak is at 409 nm. There are other photosensitisers that induce the production of compounds with other absorption spectra, such as chlorins, bacteriochlorins, methylene blue, rose bengal, etc.

Advantageously, as observed in Figures 4 and 7, the lighting device comprises at least one second LED diode (12) emitting light (8) in a second wavelength (82) different from the first wavelength (81) of the first LED diode (11), said second wavelength (82) substantially coinciding with at least one more sensitive second wavelength (82) of a photosensitiser (84) induced in a patient (7) by at least one photosensitising compound (85).

In another embodiment, at least one LED diode (1) of a first support arm (2) emits light (8) in a first wavelength (81), at least one LED diode (1) of a second support arm (2) emits light (8) in a second wavelength (82), and optionally at least one LED diode (1) of a third support arm (2) emits light (8) in a third wavelength (83), with the first, second and third wavelengths (81, 82, 83) being different from one another.

With respect to the photosensitising compounds (85), in relation to different wavelengths, the following specific embodiments are mentioned:
- The first wavelength (81) is 405 nm, the second wavelength (82) 530 nm, and the third wavelength (83) 630 nm for a porphyrin as a photosensitising compound (85), 405 nm corresponding to violet, 530 nm corresponding to green, and 630 nm corresponding to red.
- The first wavelength (81) is 405 nm, the second wavelength (82) 530 nm, and the third wavelength 660 nm for a chlorin as a photosensitising compound (85), 405 nm corresponding to violet, 530 nm corresponding to green, and 660 nm corresponding to red.
- The first wavelength (81), the second wavelength (82), and the third wavelength are 530 nm for rosa bengal as a photosensitising compound (85), 530 nm corresponding to green.
- The first wavelength (81) is 630 nm and the second wavelength (82) 660 nm for methylene blue as a photosensitising compound (85), 630 nm corresponding to red and 660 nm corresponding to red. Methylene blue would be at a preferred concentration of 31.2 µmol/L.

Optionally, for certain photosensitising compounds (85), the lighting device comprises only two support arms (2) arranged with an orientation of 180° with respect to one another. Alternatively, the control means (53) deactivate the light (8) of one of the three support arms (2), such that the support arms (2) need not be facing each other at 180° to concentrate the light (8) in a treatment point (3). For this configuration, the first wavelength (81) is 405 nm and the second wavelength (82) 790 nm for bacteriochlorin as a photosensitising compound (85), 405 nm corresponding to violet and 790 nm corresponding to infrared.

As for the photosensitising compound (85), reference is made to those shown in Table I of Figure 8. From that table, all of them can be used, but the most widely used compounds are Metvix-Metvixia, with methyl-aminolevulinic as the active ingredient, and Ameluz, with alpha-aminolevulinic as the active ingredient, both of which induce PpIX, i.e., protoporphyrin IX.

According to another aspect of the invention, as observed in Figure 1, the lighting device comprises a power source (52) and control means (53) for the power supply to the LED diodes (1).

At this point, note that in a preferred embodiment of the invention, each intermediate array (6) is controlled independently, being powered by an actuator or driver which guarantees a constant working current of 350 mA. The actuator or driver itself is what transforms the alternating current of the grid and the grid voltage into a low-voltage DC current suitable for the operation of the LED diodes (1). The adjustment means (54) have analogue and digital inputs and can be programmed via USB by means of control software running on a personal computer. The adjustment means (54) are connected to and controlled by means of relays that operate as an on/off switch for each of the actuators or drivers that supply power to each of the intermediate arrays (6) with LED diodes (1) of the lighting device.

Optionally, as observed in Figure 1, the control means (53) are configured to determine the combination of LED diodes (1) to be activated in a specific sequence of times.

Additionally, as observed in Figure 1, the lighting device comprises an interface (55) for the user (9) with adjustment means (54) for adjusting the configuration of the control means (53).

The details, shapes, dimensions and other accessory elements, as well as the components used in the implementation of the lighting device may be suitably replaced by others which are technically equivalent and do not depart from the essence of the invention and the scope defined by the claims included after the following list.

### List of reference numbers:

- 1: LED diode
- 11: first LED diode
- 12: second LED diode
- 2: support arm
- 21: segment
- 22: articulation
- 3: treatment point
- 4: light-concentrating means
- 41: collimating lens
- 51: holding means
- 52: power source
- 53: control means
- 54: adjustment means
- 55: interface
- 55a: display
- 55b: keypad
- 6: intermediate array
- 61: plane
- 62: board
- 62a: electrical connection means
- 62b: fixing means
- 7: patient
- 71: dermatological lesion
- 8: light
- 81: first wavelength
- 82: second wavelength
- 83: third wavelength
- 84: photosensitiser
- 85: photosensitising compound
- 9: user

## Claims

1. A lighting device comprising a plurality of LED diodes (1), **characterised in that** said LED diodes (1) are installed and distributed on at least one support arm (2), all the LED diodes (1) being oriented towards a single treatment point (3), and the emitted light (8) of said diodes being focussed towards said treatment point (3) by means of light-concentrating means (4).

2. The lighting device according to claim 1, **characterised in that** it comprises a plurality of support arms (2), with at least one LED diode (1) on each one.

3. The lighting device according to claim 2, **characterised in that** it comprises a plurality of LED diodes (1) in each of the support arms (2).

4. The lighting device according to any of claims 2 to 3, **characterised in that** the support arms (2) are installed on holding means (51).

5. The lighting device according to any of claims 2 to 4, **characterised in that** it comprises three support arms (2) arranged with an orientation of 120° with respect to one another.

6. The lighting device according to claim 3, **characterised in that** the LED diodes (1) are installed on each support arm (2) by means of a removable intermediate array (6).

7. The lighting device according to claim 6, **characterised in that** said intermediate array (6) comprises a plurality of planes (61) on which each of the LED diodes (1) is fixed.

8. The lighting device according to claim 7, **characterised in that** the planes (61) of the intermediate array (6) are arranged with a fixed orientation of 160° with respect to one another.

9. The lighting device according to claim 8, **characterised in that** each support arm (2) comprises a plurality of segments (21) with a fixed orientation of 160° with respect to one another, on which the planes (61) of the intermediate array (6) are seated.

10. The lighting device according to claim 7, **characterised in that** each support arm (2) comprises a plurality of segments (21), attached to one another by means of articulations (22), wherein each plane (61) of the intermediate array (6) is seated on each of the segments (21).

11. The lighting device according to any of the preceding claims, **characterised in that** at least one support arm (2) is made of aluminium.

12. The lighting device according to any of the preceding claims, **characterised in that** the light-concentrating means (4) comprise at least one collimating lens (41) for the emitted light (8) emitted by each LED diode (1).

13. The lighting device according to claim 7, **characterised in that** each LED diode (1) is installed on a printed circuit board (62) including electrical connection means (62a) and fixing means (62b) for the electrical connection and fixing to each plane (61) of the intermediate array (6).

14. The lighting device according to any of the preceding claims, **characterised in that** it comprises a power source (52) and control means (53) for the power supply to the LED diodes (1).

15. The lighting device according to claim 14, **characterised in that** the control means (53) are configured to determine the combination of LED diodes (1) to be activated in a specific sequence of times.

16. The lighting device according to claim 15, **characterised in that** it comprises an interface (55) for the user (9) with adjustment means (54) for adjusting the configuration of the control means (53).

17. The lighting device according to any of the preceding claims, **characterised in that** the LED diodes (1) are high-power LED diodes.

18. The lighting device according to claim 17, **characterised in that** it comprises at least one first LED diode (11) emitting light (8) in a first wavelength (81), said first wavelength (81) substantially coinciding with at least one more sensitive first wavelength (81) of a photosensitiser (84) induced in a patient (7) by at least one photosensitising compound (85).

19. The lighting device according to claim 18, **characterised in that** it comprises at least one second LED diode (12) emitting light (8) in a second wavelength (82) different from the first wavelength (81) of the first LED diode (11), said second wavelength (82) substantially coinciding with at least one more sensitive second wavelength (82) of a photosensitiser (84) induced in a patient (7) by at least one photosensitising compound (85).
